# EUROPÄISCHE PATENTSCHRIFT

(11) **EP 2 150 311 B1**
(45) Veröffentlichungstag und Bekanntmachung des Hinweises auf die Patenterteilung: **15.05.2013**
(21) Anmeldenummer: 08733247.4
(22) Anmeldetag: 21.04.2008
(51) Int. Cl.: A61N 1/36, A61H 39/00

(54) **GERÄT ZUR PUNKTUAL-STIMULATION**
PUNCTUAL STIMULATION APPARATUS
APPAREIL DE STIMULATION PONCTUELLE

(30) Priorität: 20.04.2007 AT 25307 U
(43) Veröffentlichungstag der Anmeldung: 10.02.2010
(73) Patentinhaber: Medizinische Universität Wien, 1090 Wien (AT)
(72) Erfinder: Szeles Josef Constantin, 1190 Wien (AT)
(74) Vertreter: Sonn & Partner Patentanwälte
(86) Internationale Anmeldenummer: PCT/AT2008/000144
(87) Internationale Veröffentlichungsnummer: WO 2008/128270

(56) Entgegenhaltungen:
- EP-A- 0 160 753
- WO-A-01/35897
- WO-A-98/05379
- DE-A1-102005 003 735
- DE-U1- 29 517 993
- US-A- 4 267 838
- US-A- 5 025 807
- US-A- 5 397 338
- SATOR-KATZENSCHLAGER S M ET AL: "The Short- and Long-Term Benefit in Chronic Low Back Pain Through Adjuvant Electrical Versus Manual Auricular Acupuncture" ANESTHESIA & ANALGESIA, Bd. 98, 2004, Seiten 1359-1364, XP002488596 ISSN: 0003-2999

## Beschreibung

Die Erfindung bezieht sich auf ein Gerät zur Punktual-Stimulation von im Bereich der Ohren liegenden Endigungen von zu Hirnstammkernen führenden Nerven, welches Gerät einen batteriegespeisten Behandlungsstromgenerator aufweist, der mit einer einen niederfrequenten Behandlungsstrom formenden Elektronikschaltung versehen ist, und welches Gerät weiter mindestens eine an einer Nervenendigung zu positionierende Elektrode aufweist, die über eine eigene flexible Leitung mit dem Behandlungsstromgenerator verbunden ist.

Es ist in einem in "Anesthesia & Analgesia" 98, (2004): 1359-1364 publizierten Aufsatz ein Gerät vorgenannter Art beschrieben, welches ein ovales Gehäuse aufweist, dessen Abmessungen etwas geringer als die Abmessungen üblicher Ohrmuscheln sind und das neben einem Ohr eines Patienten mittels Klebefixierung gehalten angeordnet wird. Ein Zugriff zu den elektrisch funktionellen Bauelementen der Elektronikschaltung, welche mit Hörhilfen-Miniaturbatterien gespeist wird, ist nicht vorgesehen und erscheint in dem an einem Patienten angebrachten Zustand auch nicht gangbar. Auch die WO 01/35897 A1 beschreibt ein derartiges Gerät.

In der DE 295 17 993 U1 ist ein Elektro-Therapiegerät beschrieben, welches zur Entwöhnung von Nikotinabhängigkeit mittels elektrischen Stromes, der durch eine zu behandelnde Person geleitet wird, dienen soll. Der Behandlungsstromgenerator und die zu dessen Speisung vorgesehenen Batterien sind in einem etwa handgroßen Kästchen untergebracht, das mit einem um den Hals der zu behandelnden Person gelegten Halsband, welches keine elektrische Funktion hat, getragen wird. An diesem Kästchen sind Steckkontakte für den Anschluss einer Handsonde einerseits und für den Anschluss von Ohrsonden andererseits vorgesehen, wobei die Ohrsonden zum Einführen in den Gehörgang und für einen Oberflächenkontakt ausgebildet sind. Eine Ohrsonde mit für einen Oberflächenkontakt ausgebildeten Elektroden ist auch bei einem in der DE 10 2005 003 735 A1 beschriebenen Gerät vorgesehen, das zur Stimulation von im Ohr verlaufenden Nerven vorgesehen ist. Sowohl dieses Gerät als auch das in der vorgenannten DE 295 17 993 U1 beschriebene Gerät sind mit von außen frei bedienbaren Einstellelementen für die Parameter des Behandlungsstromes versehen, und es sind die Ohrsonden dahingehend ausgebildet, dass sie von der zu behandelnden Person jederzeit nach Belieben in den Gehörgang eingeführt oder aus diesem herausgenommen werden können, wobei beim Einführen ein gezieltes Positionieren der Elektroden an bestimmten in räumlich engen Bereichen vorliegenden Endigungen von zu stimulierenden Nerven nicht erwartet werden kann. Dies im wesentlichen baulichen und funktionellen Gegensatz zu den Verhältnissen, die bei Geräten vorliegen, wie sie in den obgenannten Publikationen in "Anesthesia & Analgesia" und WO 01/35897 A1 beschrieben sind. Die bei diesen Geräten vorgesehenen kurzen Nadelelektroden sind von einem Arzt exakt an einer zu stimulierenden Stelle eines Nervs zu setzen und verbleiben dort für einen längeren Behandlungszeitraum, wobei auf minimal invasive Weise, bzw. praktisch nicht invasive Weise, mit hoher Treffsicherheit eine bestimmte therapeutische Wirkung erzielbar ist.

Die US-5 025 807 beschreibt ein zur Behandlung von Epilepsie und anderen motorischen Störungen vorgesehenes Stimulationsgerät mit einem in der Achselhöhle implantierten Behandlungsstromgenerator, von dem implantierte Leitungen zu gleichfalls implantierten Elektroden führen, welche an einem im Hals eines Patienten verlaufenden Bereich des Nervus vagus angeordnet sind. Es ist dabei auch eine automatische Steuerung des Behandlungsstromgenerators durch Erfassung von Hirnströmen mittels am Schädel angeordneter Elektroden in Betracht gezogen. Diese Technik ist mit einem größeren chirurgischen Aufwand und entsprechenden Risken verbunden und unterscheidet sich in mannigfacher Art von Geräten hier in Rede stehender Art.

Die WO 98/05379 beschreibt ein Verfahren und eine stationäre Vorrichtung zur Stimulationsbehandlung, wobei Behandlungsströme, die synchron zum Herzschlag verlaufen, angewendet werden. Es sind dabei für die Stimulation an einem Kopfband angeordnete Flächenelektroden oder in den Rumpf eingestochene Akupunkturnadeln vorgesehen.

Es ist ein Ziel der Erfindung, ein Gerät vorstehend genannter Art zu schaffen, welches hinsichtlich der therapeutischen Anwendungen verbesserte Eigenschaften aufweist. Es soll die Möglichkeit gegeben sein, die Stimulationstherapie durchgehend über einige Tage ohne wesentlichen Behinderung der Lebensumstände und einer allfälligen Erwerbsarbeit durchführen zu können und dabei soll die Handhabung des Gerätes einfach sein, der Stimulationsstrom soll weitgehend unabhängig gegenüber äußeren Einflüssen sein und es soll darüberhinaus ein breiter Anwendungsbereich des Gerätes ermöglicht werden.

Das erfindungsgemäße Gerät eingangs erwähnte Art ist dadurch gekennzeichnet, dass das Gerät einen in Form einer um den Hals eines Patienten zu legenden Manschette oder Bandes ausgebildeten Träger aufweist, der Speisebatterien aufnimmt, welche mindestens einen Teil der Betriebsenergie des Gerätes liefern, und der weiter mit mindestens einem Teil der für die Bildung des Behandlungsstromes vorgesehenen elektrisch wirksamen Bauteile des Gerätes versehen ist.

Das Vorsehen eines in Form einer um den Hals eines Patienten zu legenden Manschette oder Bandes ausgebildeten Trägers, der seinerseits Batterien zur Speisung des Gerätes und auch mindestens einen Teil der elektrischen Bauteile aufnimmt, die für die Bildung des Behandlungsstromes dienen, hat den Vorteil, dass diese Elemente des Gerätes einerseits in der Nähe des Ohrbereiches platziert sind und andererseits gut zugänglich sind, was zum Beispiel für einen Austausch der Batterien und für Kontrollmaßnahmen und Einstellmanipulationen für den Behandlungsstrom günstig ist. Es ist dabei eine stabile Fixierung am Körper und gewünschtenfalls ein Abdecken des Trägers mit Kleidung problemlos möglich. Beim Batterieaustausch und bei Manipulationen an den am Träger vorgesehenen elektrischen Bauteilen, wie z.B. beim Einstellen oder Ändern eines Behandlungsstromprogrammes, kann eine mechanische Beeinträchtigung der im Ohrbereich platzierten Elektroden vermieden werden. Es wird ein besonders guter Sitz des Trägers am Körper durch die Ausbildung des Trägers in Form einer Manschette oder eines Bandes erzielt. Eine solche Manschette kann gewünschtenfalls die Form eines Kragens haben, d.h. eine flexible Überdeckung aufweisen.

Als elektrisch wirksame Bauteile, die für die Bildung des Behandlungsstromes vorgesehen sind, sind neben unmittelbar im Stromkreis des Behandlungsstromes liegenden Bauteilen, wie einer Basiselektrode oder einem Halbleiterelement, das direkt mit einer im Ohrbereich platzierten Elektrode verbunden ist, auch steuernde Schaltkreise der den Behandlungsstrom formenden Elektronikschaltung zu verstehen. Ist der Träger mit einer Basiselektrode versehen, kann diese an einer kurzen flexiblen Leitung sitzen, die am Träger vorgesehen ist, so dass die Kontaktstelle, an der diese Basiselektrode an der Haut anliegt, gewählt werden kann, oder man sieht eine als Flächenelektrode ausgebildete Basiselektrode an der mit der Hautoberfläche des Patienten in Berührung zu bringenden Seite des in Form einer um den Hals eines Patienten zu legenden Manschette oder Bandes ausgebildeten Trägers vor, wobei bei einem festen Sitz des Trägers zugleich ein stabiler Andruck der Basiselektrode an die Haut des Patienten gegeben ist. Es kann auch mindestens ein Behandlungsstromgenerator, der eine den Behandlungsstrom formende Elektronikschaltung umfasst und diesen Strom über mindestens eine Elektrode einer zu stimulierenden Nervenendigung zuführt, als Ganzes an dem in Form einer um den Hals eines Patienten zu legenden Manschette oder Bandes ausgebildeten Träger vorgesehen sein, und zwar mit dem Träger fest verbunden bzw. mit dem Träger integriert, oder lösbar am Träger angebracht, wobei letztere Ausbildung hinsichtlich der Fertigung und auch hinsichtlich einer Langzeittherapie, bei der im Laufe der Zeit Änderungen der Stimulation vorgesehen sein können, Vorteile zu bieten vermag. Anderseits kann auch mindestens ein Behandlungsstromgenerator im Bereich der Ohren angeordnet sein und mit elektrisch wirksamen Bauteilen, die sich am Träger befinden, über flexible Leitungen in Verbindung stehen. Solche Leitungen können z.B. eine Verbindung zu einer am Träger vorgesehenen Basiselektrode oder eine Verbindung zu am Träger vorgesehenen Speisebatterien oder eine Verbindung zu Schaltkreisen bilden, welche auf die im Behandlungsstromgenerator vorgesehene Elektronikschaltung steuernd einwirken. Derartige Schaltkreise können am Träger gut zugänglich angeordnet werden, so dass Justierungen oder verändernde Einstellungen, die den Behandlungsstrom beeinflussen sollen, ebenso wie Überwachungen der in diesen Schaltkreisen fließenden Signale durch anzuschließende Geräte und ebenso wie die Zuleitung externer Signale, die von Körperfunktionssensoren stammen können, problemlos vorgenommen werden können. Soweit es sich nur um die Übertragung von Steuersignalen vom am Träger vorgesehenen Schaltkreisen zu einer Elektronikschaltung eines im Ohrbereich angeordneten Behandlungsstromgenerators handelt, kann vorteilhaft als Verbindung auch eine drahtlose Verbindung in Form einer Sender-Empfänger-Strecke vorgesehen werden.

Körperfunktionssensoren, wie Sensoren für Herzaktionspotentiale, Sensoren für den Puls oder Sensoren für Hirnströme, können in mechanischer und elektrischer Hinsicht stabil an am Träger befindliche Schaltkreise, die eine aus den Ausgangssignalen solcher Sensoren Steuersignale bildende Schaltung beinhalten, angeschlossen werden.

Mit dem erfindungsgemäß ausgebildeten Gerät können auf praktisch nicht-invasive Weise durch Stimulation von im Bereich der Ohren liegenden Nervenendigungen verschiedene Körperfunktionen, wie z.B. die Pulsfrequenz und der Blutdruck, beeinflusst werden, wobei durch Steuerung der Stimulation eine gezielte Veränderung der Parameter der Körperfunktionen, insbesondere im Sinne einer Normalisierung, herbeigeführt werden kann. Diese Beeinflussung erfolgt vorzugsweise unter Stützung auf Signale, die von den hinsichtlich ihrer Parameter zu verändernden Körperfunktionen hergeleitet werden. Eine diesbezügliche bevorzugte Ausführungsform des Gerätes ist dadurch gekennzeichnet, dass der in Form einer um den Hals eines Patienten zu legenden Manschette oder Bandes ausgebildete Träger, mit mindestens einem elektrische Ausgangssignale abgebenden Sensor für Körperfunktionen eines Patienten versehen ist, weiter elektronische Schaltkreise aufweist, die aus dem Ausgangssignal des Körperfunktionssensors Steuersignale bilden, welche für eine von den Körperfunktionen abhängige Steuerung mindestens eines Behandlungsstromgenerators vorgesehen sind, und weiter eine zu mindestens einem Behandlungsstromgenerator führende elektrische Signalverbindung zur Beeinflussung des Behandlungsstromes durch die von den Ausgangssignalen des Sensors hergeleiteten Steuersignale vorgesehen ist.

Eine vorteilhafte Weiterbildung dieser Ausführungsform ist dadurch gekennzeichnet, dass die elektronischen Schaltkreise, die aus dem Ausgangssignal des Körperfunktionssensors Steuersignale für den Behandlungsstromgenerator bilden, eine vom periodischen Ausgangssignal eines Körperfunktionssensors triggerbare, hinsichtlich ihrer Verzögerungszeit einstellbare Zeitverzögerungsstufe aufweisen, wobei die Zeitverzögerungsstufe ihrerseits den Behandlungsstromgenerator ansteuert, welcher an mindestens eine zur Platzierung an einer im Bereich des äußeren Ohres eines Patienten liegenden Nervenendigung bestimmte in Form einer kurzen Nadel ausgebildete Elektrode einen periodischen Behandlungsstrom abgibt, dessen Folgefrequenz der Folgefrequenz des Ausgangssignals des Körperfunktionssensors entspricht und der aus Stromflusspaketen gebildet ist, deren Länge geringer ist, als die Periodendauer des Ausgangssignals des Körperfunktionssensors, wobei die elektronischen Schaltkreise eine Korrekturstufe beinhalten, welche aus dem Frequenzwert oder aus der Kurvenform oder aus dem Amplitudenwert des Ausgangssignals des Körperfunktionssensors ein Korrektursignal bildet, das an einen die Zeitverzögerung beeinflussenden Eingang der Zeitverzögerungsstufe angeschlossen ist. Es kann mit diesem Gerät durch den Behandlungsstrom ein Einfluss auf die Parameter einer periodischen Körperfunktion, z.B. Pulsfrequenz oder Blutdruck, ausgeübt werden, wobei durch Einstellung des Zeitabstandes in Bezug auf einen Triggerzeitpunkt, z.B. die R-Zacke im Herzaktionspotential, das Ausmaß und die Art der Parameterbeeinflussung wählbar ist. Es wird die sich aus der Stimulation ergebende Veränderung der Parameter von der Korrekturstufe erfasst und das von der korrekturstufe abgegebene Korrektursignal beeinflusst, analog wie das Ist-Wert-Signal einer Regelstrecke, das Ausmaß der Zeitverzögerung der Zeitverzögerungsstufe im Sinne des Erreichens des angestrebten Ausmaßes der Veränderung der Parameter der jeweils in Betracht stehenden Körperfunktion. In der Regel soll die angestrebte Veränderung der Parameter einer periodischen Körperfunktion langsam erfolgen und es ist hiezu eine langsame Veränderung der Einstellung des vorerwähnten Zeitabstandes vorzunehmen. Dies kann individuell durch manuelle Betätigung entsprechender Einstellelemente ausgeführt werden.

Eine vorteilhafte Weiterbildung des Gerätes sieht vor, dass an die Zeitverzögerungsstufe ein Verschiebungssignalgeber angeschlossen ist, der der Zeitverzögerungsstufe ein Schiebesignal zuführt, durch das die Verzögerungszeit, im Verhältnis zur Folgefrequenz des Ausgangssignals des Körperfunktionssensors langsam, veränderbar ist. Hiedurch kann der Ablauf der Veränderung der vorgenannten Paramter auf einfache Weise über längere Zeiträume, z.B. über Stunden oder Tage, erstreckt werden, ohne dass es zwischenzeitlich einer Bedienung bedarf, so dass die Möglichkeit einer von der Therapie weitgehend unbehinderten Lebensführung besteht.

Es ist für das Ansetzen der mindestens einen stimulierenden Elektrode des Gerätes an eine im Bereich des äußeren Ohres liegende Nervenendigung von Vorteil, die zur Elektrode führende flexible Leitung mechanisch möglichst nachgiebig auszubilden und diese Leitung im Interesse einer geringen mechanischen Störungsanfälligkeit im Therapiezeitraum auch möglichst kurz zu halten. Zur einfachen Realisierung kann man eine vom Träger bis in Ohrnähe reichende mechanisch stabile Leitung vorsehen, von der ausgehend dann eine kurze besonders flexible Leitung zur betreffenden Elektrode führt.

Eine bevorzugte Ausführungsform ist dadurch gekennzeichnet, dass das Gerät mindestens eine an einer Nervenendigung im Bereich des äußeren Ohres zu positionierende Elektrode aufweist, die in Form einer kurzen Nadel ausgebildet ist, und deren flexible Leitung von einem passend in den äußeren Gehörgang einfügbaren Einsatzkörper ausgeht, der seinerseits in elektrischer Verbindung mit am in Form einer um den Hals eines Patienten zu legenden Manschette oder Bandes ausgebildeten Träger befindlichen Teilen des Gerätes steht. Man kann dazu eine mechanisch stabile flexible Leitung vom Träger zum Einsatzkörper führen und von diesem, eine Abstützung bildenden, Einsatzkörper, eine zur jeweils betreffenden Elektrode verlaufende besonders nachgiebige flexible Leitung vorsehen.

In Weiterbildung der den genannten Einsatzkörper aufweisenden Ausführungsform kann man vorteilhaft vorsehen, dass der Einsatzkörper als Gehäuse ausgebildet ist, in dem mindestens der den Behandlungsstrom an die in Form einer kurzen Nadel ausgebildeten Elektrode abgebende Teil der Elektronikschaltung des Behandlungsstromgenerators angeordnet ist.

Eine weitere Entwicklung dieser Weiterbildung ist dadurch gekennzeichnet, dass mindestens ein Behandlungsstromparameter, wie z.B. das Stromfluss-Pausen-Programm oder die Frequenz, die Amplitude und die Kurvenform des Behandlungsstromes formender Teil der Elektronikschaltung des Behandlungsstromgenerators in dem in Form einer um den Hals eines Patienten zu legenden Manschette oder Bandes ausgebildeten Träger angeordnet ist und über eine elektrische Verbindung den im Einsatzkörper befindlichen Teil der Elektronikschaltung des Behandlungsstromgenerators steuert.

Es ergibt sich so einerseits eine gute Zugänglichkeit der die Parameter des Behandlungsstromes bestimmenden Teile der am Träger befindlichen Elektronikschaltung zur Einstellung und Überwachung und andererseits eine kleine, wenig sichtbare und gut sitzende Ausbildung der im Ohrbereich vorzusehenden Teile des Gerätes. Die elektrische Verbindung vom Träger zum Einsatzkörper kann in Form einer flexiblen Leitung oder derart vorgesehen werden, dass die elektrische Verbindung drahtlos, als Sender-Empfänger-Strecke, ausgeführt ist und im Einsatzkörper eine Batterie zur Speisung des im Einsatzkörper befindlichen Teiles der Elektronikschaltung angeordnet ist.

Eine Variante zu dem mit einem Einsatzkörper versehenen Gerät ist dadurch gekennzeichnet, dass das Gerät mindestens eine an einer Nervenendigung im Bereich des äußeren Ohres zu positionierende Elektrode aufweist, deren flexible Leitung von einem außen am Ohr zu tragenden Gehäuse ausgeht, in dem mindestens der den Behandlungsstrom an die Elektrode abgebende Teil der Elektronikschaltung des Behandlungsstromgenerators angeordnet ist, und dass mindestens ein Behandlungsstromparameter, wie z.B. das Stromfluss-Pausen-Programm oder die Frequenz, die Amplitude und die Kurvenform des Behandlungsstromes formender Teil der Elektronikschaltung des Behandlungsstromgenerators in dem in Form einer um den Hals eines Patienten zu legenden Manschette oder Bandes ausgebildeten Träger angeordnet ist und über eine elektrische Verbindung den im am Ohr zu tragenden Gehäuse befindlichen Teil der Elektronikschaltung des Behandlungsstromgenerators steuert. Auch hierbei kann die zwischen dem Träger und dem außen am Ohr zu tragenden Gehäuse vorgesehene elektrische Verbindung entweder durch eine flexible Leitung oder drahtlos als Sender-Empfänger-Strecke ausgeführt sein, wobei in dem außen am Ohr zu tragenden Gehäuse eine Batterie zur Speisung des in diesem Gehäuse befindlichen Teiles der Elektronikschaltung angeordnet ist.

Im Rahmen der Anwendung des erfindungsgemäßen Gerätes sind auch spezielle Ausbildungen der wie oben ausgeführt praktisch nicht-invasiven Punktual-Stimulation von im Bereich der Ohren liegenden Nervenendigungen ins Auge gefasst, wobei außer dem Gebiet der Schmerzbekämpfung insbesondere auch die Regulation von Herzschlagfrequenz und Blutdruck und eine funktionelle Neurostimulation in Betracht gezogen sind.

So können zur Regulierung normabweichender Werte der Herzschlagfrequenz und/oder des Blutdruckes im Ohrbereich liegende Enden von Neuronen des Tractus Solitarius mit Gruppen aufeinanderfolgender elektrischer Impulse, die mit einem hinsichtlich des Startzeitpunktes der Impulsgruppen einstellbaren Behandlungsstromgenerator erzeugt werden, stimuliert werden, wobei diese Stimulation im Takt mit dem Herzschlagzyklus vorgenommen wird und hiezu mit einem am Patientenkörper angeordneten Sensor für elektrische Herzaktionspotentiale ein Elektrokardiogrammsignal gebildet wird, das einer Triggerschaltung zugeführt wird, die auf das Auftreten eines vorbestimmen Wertes im Elektrokardiogrammsignal anspricht und eine im Behandlungsstromgenerator vorgesehene einstellbare Zeitverzögerungsstufe auslöst, die nach Ablauf der jeweils eingestellten Verzögerungszeit die Abgabe einer Impulsgruppe startet, wobei der Startzeitpunkt der einzelnen Impulsgruppen durch Einstellung der Verzögerungszeit, in Bezug auf die R-Zacke des Elektrokardiogrammsignals zeitlich verschoben, im systolischen Bereich des Herzschlagzyklus vorgesehen wird, und am Beginn einer Regulierungsbehandlung zunächst ein Basiswert der Verzögerungszeit eingestellt wird und darauffolgend eine langsame Veränderung der Verzögerungszeit zur Annäherung der zu regulierenden Werte an Normen vorgenommen wird. Es kann dabei durch geeignete Einstellung der Verzögerungszeit eine Phasenverschiebung des Herzschlagzyklus in Bezug auf die vom Behandlungsstromgenerator abgegebene Impulsgruppenfolge herbeigeführt werden, die eine Veränderung der Herzschlagfrequenz im Sinne der angestrebten Regulierung erzielt lässt. Auch eine Regulierung des Blutdruckes kann durch eine derartige Einstellung der vom Behandlungsstromgenerator abgegebenen Impulsgruppenfolge erzielt werden. Durch die langsame Veranderung der Verzögerungszeit erfolgt eine allmähliche Hinführung der Herzschlagfrequenz und/oder des Blutdruckes zu den beabsichtigten Werten, wobei bei hinreichend geringer Geschwindigkeit dieser Veränderung und bei einer über Stunden und Tage durchgefuhrten Stimulation auch nach dem Ende einer solchen Behandlung die damit erzielten Werte der Herzschlagfrequenz und/oder des Blutdruckes über längere Zeit erhalten bleiben.

Es kann dabei die langsame Veränderung der Verzögerungszeit unter Berücksichtigung von durch die Stimulation aufgetretenen Veränderungen der zu regulierenden Werte vorgenommen werden. Die erwähnte Veränderung der Verzögerungszeit kann durch Zugriff auf entsprechende Einstellorgane, z.B. von einem Therapeuten, individuell gesteuert werden. Im Hinblick auf den positiven Einfluss langdauernder Stimulation ist es oft vorteilhaft, wenn die langsame Veränderung der Verzögerungszeit mit einer selbsttätig arbeitenden elektronischen Verschiebungsstufe vorgenommen wird.

Als Basiswert der Verzögerungszeit, den die Abgabe der stimulierenden Impulsgruppe bezüglich der R-Zacke des Elektrokardiogrammsignals aufweist, kann dabei vorteilhaft eine Verzögerungszeit zwischen 150 ms und 350 ms eingestellt werden.

Vorteilhaft wird vorgesehen, dass die Impulsgruppen aus Folgen von Impulsen bestehen, welche in Bezug auf eine Basiselektrode in abwechselnder Aufeinanderfolge verschiedene Polarität haben und mit einer Frequenz von 70 bis 110 Hz aufeinanderfolgen. Es ist dabei günstig, wenn die einzelnen Impulsgruppen je 4 bis 10 Impulse beinhalten. Die einzelnen Impulse weisen vorteilhaft eine Länge zwischen 0,5 und 2 ms auf. Vorteilhaft wird die Stimulation in einer Abfolge von je 15 min bis mehrere Stunden dauernden Zeitintervallen, welche mit annähernd gleichlangen Pausen abwechseln, vorgenommen. Dabei ist es auch im Sinne des Erzielens einer über die Zeit der Behandlung hinaus erhaltenen Wirkung günstig, wenn der Basiswert der Verzögerungszeit am Anfang der einzelnen Zeitintervalle, in denen eine Stimulation vorgenommen wird, der am Ende des vorhergehenden Zeitintervalls eingestellt gewesenen Verzögerungszeit angeglichen wird. Weiter wird vorzugsweise vorgesehen, dass die Stimulation gleichzeitig im Bereich beider Ohren synchron zueinander transkutan vorgenommen wird.

Im Rahmen der Anwendung des erfindungsgemäßen Gerätes kann auch eine funktionelle Neurostimulation vorgesehen werden. Es werden dabei im Bereich der Ohren liegende Endigungen von Nerven transkutan mit niederfrequenten Strömen stimuliert, welche aus Impulsgruppen bestehen, die mit zeitlichem Abstand aufeinanderfolgen. Die einzelnen Impulsgruppen ihrerseits bestehen je aus einer Reihe von Impulsen, die mit einer Frequenz zwischen 10 und 200 Hz aufeinanderfolgen, wobei die Dauer der einzelnen Impulsgruppen zwischen etwa 50 ms und 2 s liegt. Der zeitliche Abstand der Impulsgruppen voneinander liegt zweckmäßig zwischen 200 ms und 20 s. Im Rahmen dieser Vorgaben kann auch die Dauer der einzelnen Impulse gewählt werden, wobei für viele Anwendungen Zeiten zwischen 0,5 ms und 2 ms geeignet sind. Die Stimulationen können an Nervenendigungen, die im Bereich beider Ohren liegen, oder an Nervenendigungen, die im Bereich eines Ohres liegen, vorgenommen werden. Die Impulse können von gleicher Polarität sein oder abwechselnd verschiedene Polaritäten haben.

Es wurden zur Schmerzbeeinflussung mit einem erfindungsgemäßen Gerät Stimulationen der sensorischen A-Delta-Nervenfasern und der sensorischen C-Nervenfasern vorgenommen, wobei mit Impulsgruppen stimuliert wurde, die je aus 100 einzelnen Impulsen bestanden, welche mit einer Frequenz von 100 Hz aufeinanderfolgten, und die einzelnen Impulsgruppen einen Abstand von 10 s hatten. Diese Stimulation wurde 15 min lang durchgeführt; 90 Impulsgruppen. Es wurde damit eine etwa 2 Stunden andauernde Analgesie erzielt.

Weiter wurden Stimulationen mit Behandlungsströmen vorgenommen, welche aus Impulsgruppen von je drei aufeinanderfolgenden Impulsen bestanden. Die drei Impulse jeder Impulsgruppe folgten mit einer Frequenz von 50 Hz aufeinander, sodass die Dauer der einzelnen Impulsgruppen bei 60 ms lag. Der zeitliche Abstand der Impulsgruppen voneinander war 200 ms. Nach einer 15 min dauernden Stimulation ergab sich eine etwa 1 Stunde anhaltende Supprimierung der motorischen Funktion der Gehirnrinde. Es wurde auch eine Variante dieser Stimulation, bei der aus drei Impulsen bestehende Impulsgruppen vorgesehen sind, vorgenommen, wobei auf jeweils 10 Impulsgruppen eine Pause von 10 s folgte und diese Abfolge längere Zeit fortgesetzt wurde, wobei während dieser Zeit die motorische Funktion der Gehirnrinde supprimiert wurde. Es ist damit ein vorteilhaftes funktionelles Einwirken auf verschiedene Bereiche des Gehirns auf nicht-invasivem bzw. minimalinvasivem (transkutanem) Weg möglich, wobei so eine positive Beeinflussung neurologischer Erkrankungen, wie Epilepsie, Alzheimer oder Parkinson, ermöglicht wird.

Im Rahmen der Anwendung des erfindungsgemäßen Gerätes kann auch für eine funktionelle Neurostimulation vorgesehen werden, dass im Bereich der Ohren liegende Enden von Neuronen des Tractus Solitarius während der Systole jedes Herzschlagzyklus und im Bereich der Ohren liegende Enden von Neuronen des Lucus LC während der Diastole jedes Herzschlagzyklus mit elektrischen Impulsen, die von einem Behandlungsstromgenerator erzeugt werden, stimuliert werden, wobei zur Synchronisation der stimulierenden Impulse mit dem Herzschlagzyklus mit einem auf die elektrischen Herzaktionspotentiale ansprechenden Sensor ein Elektrokardiogrammsignal gebildet wird, das einer Triggerschaltung zugeführt wird, die auf das Auftreten eines vorbestimmten Wertes im Elektrokardiogrammsignal anspricht und im Behandlungsstromgenerator vorgesehene Zeitverzögerungsstufen auslöst, die nach Ablauf von Verzögerungszeiten die Abgabe von stimulierenden Impulsen während der Systole und während der Diastole starten.

Hierbei kann man auch vorsehen, dass die Stimulation an Neuronen des Tractus Solitarius an einem der Ohren eines Patienten und die Stimulation an Neuronen des Lucus LC am anderen der Ohren dieses Patienten vorgenommen wird. Dies lässt eine Intensivierung der Wirkung erzielen.

Die Erfindung wird nun an Hand von Ausführungsbeispielen unter Bezugnahme auf die Zeichnung, in der solche Ausführungsbeispiele schematisch dargestellt sind, weiter erläutert.

In der Zeichnung zeigt
Fig. 1 ein erstes Ausführungsbeispiel eines erfindungsgemäß ausgebildeten Gerätes in einer schematisierten Ansicht,
Fig. 2 den Träger dieses Gerätes in einem Schnitt gemäß der Linie II-II in Fig. 1,
Fig. 3 eine Blockschaltung der elektrischen Bauteile dieses Gerätes und
Fig. 4 Funktionsdiagramme, wie sie in einzelnen Stufen der in Fig. 3 dargestellten Blockschaltung auftreten;
Fig. 5 zeigt eine weitere Ausführungsform eines erfindungsgemäß ausgebildeten Gerätes in einer schematisierten Ansicht und Fig. 6 eine Blockschaltung dieses Ausführungsbeispiels;
Fig. 7 zeigt eine Blockschaltung einer weiteren Ausführungsform eines erfindungsgemäß ausgebildeten Gerätes;
Fig. 8 zeigt eine Blockschaltung einer Ausführungsform eines erfindungsgemäß ausgebildeten Gerätes.

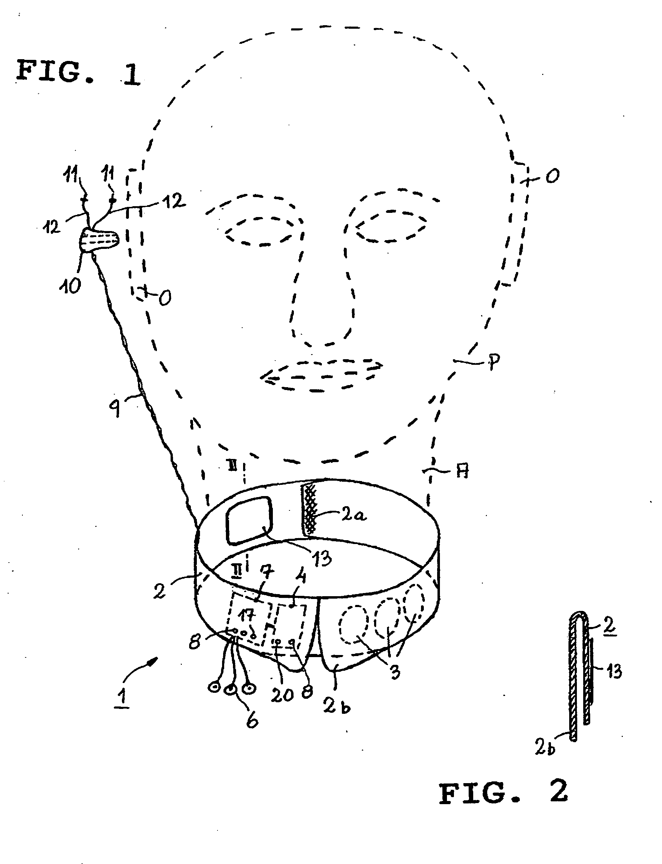

Fig. 1 zeigt in schematisierter Ansicht eine Ausführungsform eines erfindungsgemäß ausgebildeten Gerätes 1, welches einen Träger 2 aufweist, der dazu vorgesehen ist, im Bereich des Halses A eines Patienten P angeordnet zu werden, dessen Kopf-HalsBereich in Fig. 1 strichliert angedeutet ist. Das Gerät 1 ist zur Punktual-Stimulation von im Bereich der Ohren O liegenden Endigungen von zu Hirnstammkernen führenden Nerven vorgesehen und weist einen batteriegespeisten Behandlungsstromgenerator auf, der kleinen Elektroden 11, die an den genannten Nervenendigungen zu platzieren sind, einen niederfrequenten Behandlungsstrom zuführt. Der Behandlungsstromgenerator 4 steht in Verbindung mit elektronischen Schaltkreisen 7 und kann mit diesen einstückig oder in Form mehrerer zusammengeschalteter Bauteile ausgeführt sein und es sind der Behandlungsstromgenerator 4 und die elektronischen Schaltkreise 7 im Träger 2, der in Form einer um den Hals A des Patienten P zu legenden Manschette ausgebildet ist, angeordnet. Von dem einen Behandlungsstromgenerator 4 führt eine mechanisch stabile flexible Leitung 9 zu einem in ein Ohr des Patienten einsteckbaren Einsatzkörper 10, von dem seinerseits besonders nachgiebige flexible Leitungen 12, die zu den Elektroden 11 führen, ausgehen. Die Elektroden 11 sind in Form kurzer Nadeln ausgebildet, die dazu vorgesehen sind, auf transkutanem Wege die im Ohrbereich dicht unter der Haut liegenden Nervenendigungen zu kontaktieren. Der Einsatzkörper 10 ist durchbohrt, damit die Hörwahrnehmung nicht beeinträchtigt wird. Der Einsatzkörper kann auch weggelassen werden, wobei die besonders nachgiebigen flexiblen Leitungen 12 direkt an das Ende der stabilen flexiblen Leitung 9 angeschlossen werden. Der Träger 2 ist mit einer Basiselektrode 13 versehen, welche beim Herumlegen des manschettenartig ausgebildeten Trägers mit der Hautoberfläche des Patienten in Berührung gebracht wird, wobei durch das Schließen der Manschette, für das z.B. ein Klettverschluss 2a vorgesehen sein kann, eine innige Anlage der Basiselektrode 13 an die Hautoberfläche des Patienten erreicht wird. Über die Basiselektrode 13 wird der über die Elektroden 11 und über mindestens eine Nervenendigung, die sich im Bereich der Ohren des Patienten befindet, führende Behandlungsstromkreis geschlossen. Der Träger 2 nimmt weiter Speisebatterien 3 auf, welche die Betriebsenergie des Gerätes 1 liefern.

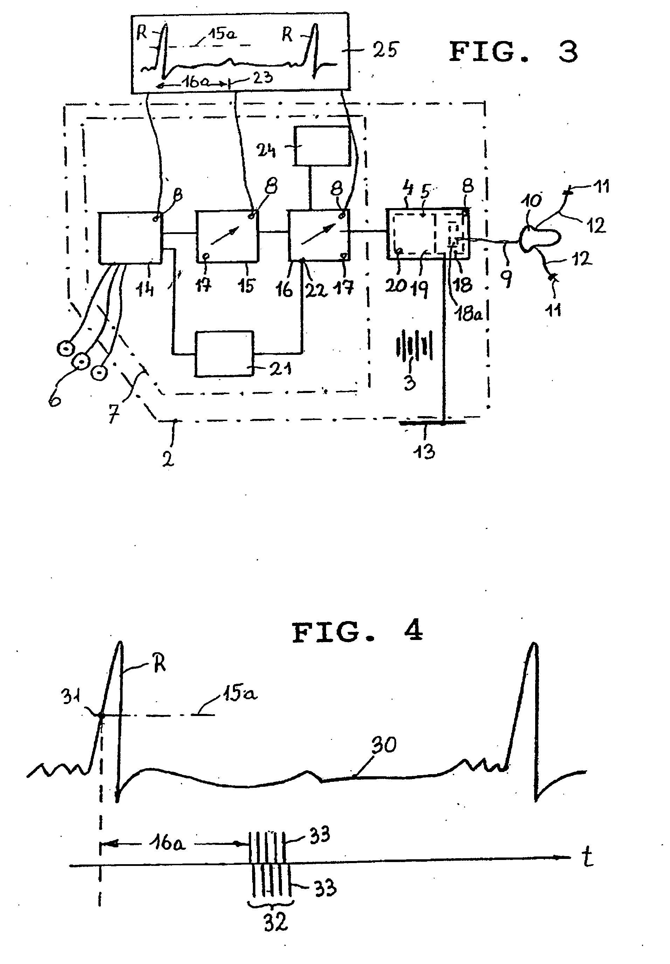

Der Behandlungsstrom, der über die Elektroden 11 Nervenendigungen zugeführt wird, welche im Bereich der Ohren des zu behandelnden Patienten dicht unter der Haut liegen, um über diese Nervenendigungen eine Stimulation vorzunehmen, besteht aus einer niederfrequenten Folge von Impulsen. Diese Impulsfolge wird mit einer periodisch ablaufenden Körperfunktion des Patienten, im vorliegenden Fall mit der Herzschlagfunktion, koordiniert. Es ist hiezu im dargestellten Fall ein aus mehreren Elektroden gebildeter Sensor 6 für elektrische Herzaktionspotentiale vorgesehen, der, wie die schematische Blockschaltung in Fig. 3 zeigt, an einen Sensorverstärker 14 angeschlossen ist. Das darin verstärkte Signal gelangt zu einem einstellbaren Trigger 15, der jeweils beim Auftreten eines bestimmten Spannungswertes im sich wiederholenden Zyklus des Herzaktionspotentials einen Steuerimpuls an eine dem Trigger 15 nachgeschaltete Zeitverzögerungsstufe 16 abgibt, welche ihrerseits mit entsprechender zeitlicher Verzögerung nach dem Auftreten eines Triggerimpulses den Behandlungsstromgenerator 4 steuert. Im Behandlungsstromgenerator 4 ist eine mit einem Mikroprozessor versehene Elektronikschaltung 5 vorgesehen, in deren erstem Teil 19 der durch aufeinanderfolgende Impulse gebildete Behandlungsstrom, der den vorerwähnten Nervenendigungen zugeführt wird, hinsichtlich Wellenform und Amplitude sowie hinsichtlich der Zeitdauer von Impulsen und dazwischenliegenden Impulspausen und der Zeitdauer einzelner Impulsfolgen und dazwischenliegender Pausen, in denen kein Behandlungsstrom fließt, festgelegt wird. Der Teil 19 der Elektronikschaltung 5 steuert eine Ausgangsstufe 18 des Behandlungsstromgenerators, in der eine Stromkonstanzschaltungsanordnung 18a vorgesehen ist, welche den Behandlungsstrom gegenüber allfällig unbeabsichtigt auftretenden Widerstandsänderungen im Elektroden-Patienten-Stromkreis stabilisiert.

An den einzenen Funktionsstufen im Rahmen der elektronischen Schaltkreise 7, nämlich am Sensorverstärker 14, am Trigger 15, an der Zeitverzögerungsstufe 16 und am Behandlungsstromgenerator 4 sind Anschlussstellen 8 vorgesehen, an welche ein Display 25 zur Darstellung des Verlaufes der in diesen Funktionsstufen auftretenden Potentiale anschließbar ist. Es ist dabei wie in Fig. 3 angedeutet ist, von der am Sensorverstärker vorgesehenen Anschlussstelle 8 der Verlauf der vom Sensor 6 erfassten Herzaktionspotentiale abnehmbar und man kann durch Einblenden des jeweils eingestellten Wertes der Triggerschaltschwelle 15a jene Stelle im Verlauf des Herzaktionspotentials sichtbar machen, an der ein Triggerimpuls an die Zeitverzögerungsstufe 16 abgegeben wird. Es kann dann weiter im Display 25 der Zeitabstand 16a eingeblendet werden, der zwischen dem Ansprechen des Triggers 15 und der Abgabe eines Steuersignals 23 an den Behandlungsstromgenerator 4 liegt, so dass aus dem Display 25 ersehen werden kann, in welchem Zeitpunkt des Herzaktionszyklus eine Stimulation an den im Ohrbereich liegenden Nervenendigungen erfolgt. Zur Festlegung des Triggerzeitpunktes kann vorteilhaft, wie in Fig. 3 angedeutet, die R-Zacke, welche im Verlauf des Herzaktionspotentials auftritt, gewählt werden.

Durch die hier in Rede stehende Stimulation kann der Ablauf der vom Sensor 6 erfassten Körperfunktion beeinflusst werden, wobei die zeitliche Position der Stimulation im Zyklus des vom Sensor 6 erfassten Potentialablaufes von Bedeutung ist. Diese zeitliche Position ergibt sich aus dem Ausmaß der Zeitverzögerung in der Zeitverzögerungsstufe 16 und kann dadurch, dass die Zeitverzögerungsstufe 16 einstellbar ausgebildet ist, variiert werden. Da verschiedene Patienten in verschieden großem Ausmaß auf eine solche Stimulation ansprechen, ist es vorteilhaft, das Ansprechverhalten des jeweiligen Patienten in die Einstellung der Zeitverzögerungsstufe 16 einzubeziehen und es ist hiezu eine Korrekturstufe 21 vorgesehen, welche aus einem jeweils im Herzaktionspotential vorliegenden Ist-Wert, z.B. der Herzschlagfrequenz, ein Korrektursignal bildet und dieses Korrektursignal wird einem an der Zeitverzögerungsstufe 16 vorgesehenen Eingang 22 zugeführt, über den das Ausmaß der Zeitverzögerung, die in der Zeitverzögerungsstufe 16 erfolgt, veränderbar ist.

Wie erwähnt, sind die einzelnen Funktionsstufen der elektronischen Schaltkreise 7, nämlich der Trigger und die Zeitverzögerungsstufe sowie auch der Behandlungsstromgenerator 4 hinsichtlich ihrer Funktionseigenschaften einstellbar bzw. programmierbar und es sind hiezu für die erwähnten Funktionsstufen Anschlussstellen 17 für die Zufuhr von Einstellsignalen und eine Anschlussstelle 20 zur Zufuhr eines Programmiersignals an den Behandlungsstromgenerator 4 vorgesehen. Solche Anschlussstellen, wie auch die Anschlussstellen 8, können in Form einfacher Steckkontakte realisiert sein oder in Form von Einrichtungen mit Sende-Empfangs-Techniken, die in Form induktiver Koppelglieder oder z.B. mit Bluetooth-Techniken aufgebaut sein können.

Um die Möglichkeit zu schaffen, im Rahmen einer sich über längere Zeiträume erstreckenden Stimulationsbehandlung eine allmähliche Veränderung des Zeitabstandes 16a vorzunehmen, der zwischen dem Triggerzeitpunkt und der Ansteuerung des Behandlungsstromgenerators liegt, kann vorteilhaft ein Verschiebesignalgeber 24 vorgesehen werden, der der Zeitverzögerungsstufe 16 ein das Ausmaß der Zeitverzögerung langsam veränderndes Signal zuführt.

Es ist vorstehend bei der Beschreibung der in Fig. 3 dargestellten Schaltungsanordnung der Einsatz eines Sensors 6 angesprochen, der elektrische Herzaktionspotentiale aufnimmt. Es können bei einem solchen Gerät auch andere Sensoren, welche veränderliche Körperfunktionen von Patienten erfassen, vorgesehen werden, wie z.B. Pulssensoren oder Sensoren, welche Hirnströme erfassen. Auch Blutdrucksensoren können ergänzend vorgesehen werden.

Fig. 4 zeigt in Form eines Zeitdiagrammes die bei einer vorteilhaften Ausführungsform der Stimulation vorgesehenen zeitlichen Zusammenhänge zwischen dem als Bezugsgröße vorgesehenen Verlauf des elektrischen Herzaktionspotentials und dem Behandlungsstrom, wobei in der oberen Zeile der Fig. 4 der Verlauf des Herzaktionspotentials und in der unteren Zeile der Verlauf des Behandlungsstromes schematisch dargestellt ist. Auf der das Herzaktionspotential 30 darstellenden Kurve ist der sich aus der Triggerschaltschwelle 15a ergebende Triggerzeitpunkt mit 31 bezeichnet und liegt im Anstieg der R-Zacke. Mit einer Verzögerungszeit 16a von 150 bis 350 ms in Bezug auf den Triggerzeitpunkt 31 wird der Behandlungsstromgenerator angesteuert und gibt je Herzaktionszyklus eine Impulsgruppe 32 an die im Ohrbereich positionierten Stimulationselektroden ab. Diese Impulsgruppen 32 bestehen je aus 4 bis 10 Impulsen 33, welche in Bezug auf eine Basiselektrode in abwechselnder Aufeinanderfolge verschiedene Polarität haben und mit einer Frequenz von 70 bis 110 Hz aufeinanderfolgen. Die einzelnen Impulse 33 weisen dabei eine Länge zwischen 0,5 und 2 ms auf.

Wie aus den Figuren 1 und 2 ersehen werden kann, weist der Träger 2 an seiner Außenseite eine flexible kragenartig geformte Überdeckung 2b auf, welche einfach hochgeklappt werden kann, wonach die Anschlüsse 8, 17, 20 für die Kontrolle und für die Vornahme von Einstellungen oder Programmierungen frei zugänglich sind und die Speisebatterien 3 im Bedarfsfall leicht ausgetauscht werden können. Die Überdeckung 2b kann gewünschtenfalls auch modisch ausgebildet werden.

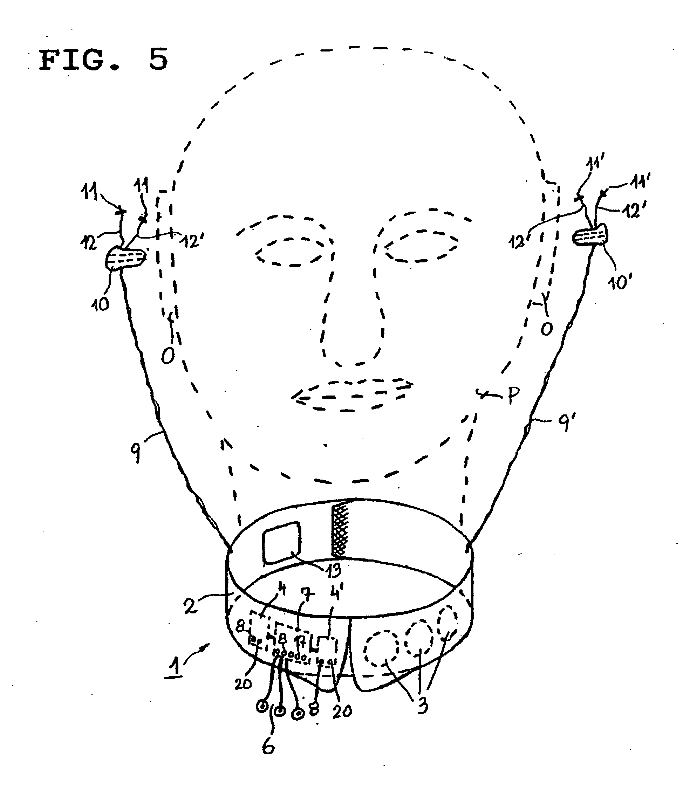

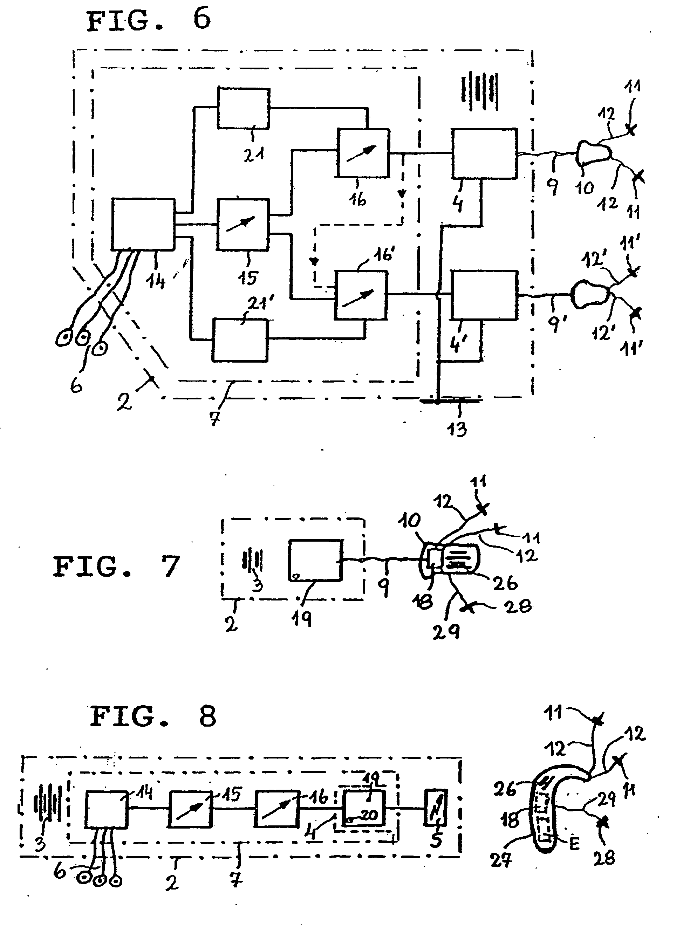

Es ist für verschiedene Einsatzgebiete der hier in Rede stehenden Stimulationsbehandlung sachdienlich, eine Stimulation an Nervenendigungen im Bereich beider Ohren vom Patienten vorzunehmen. Je nach Einsatzfall kommt dabei eine simultane Zufuhr von niederfrequenten impulsförmigen Stimulationsströmen an im Bereich beider Ohren liegende Nervenendigungen in Betracht oder eine Stimulation, bei der die impulsformigen Stimulationsströme, welche an Nervenendigungen zugeführt werden, welche im Bereich des einen Ohres des Patienten liegen, in Bezug auf impulsförmige Stimulationsströme, welche Nervenendigungen zugeführt werden, die im Bereich des anderen Ohres eines Patienten liegen, zeitlich verschoben sind. Zur Realisierung solcher Therapiemöglichkeiten ist die in den Figuren 5 und 6 dargestellte Ausführungsform eines erfindungsgemäß ausgebildeten Gerätes vorgesehen. Dieses Gerät 1 weist, ähnlich wie das in den Figuren 1 bis 4 dargestellte Gerät, einen in Form einer Manschette ausgebildeten Träger 2 auf, an dem elektronische Schaltkreise 7, Behandlungsstromgeneratoren 4, 4' und eine Basiselektrode 13 angeordnet sind und der Speisebatterien 3 für den Betrieb des Gerätes aufnimmt. Die elektronischen Schaltkreise 7 beinhalten einen Sensorverstärker 14, an den ein mit mehreren Elektroden versehener Körperfunktionssensor 6 angeschlossen ist, sowie einen Trigger 15, der zwei Ausgänge aufweist, wobei an den einen Ausgang eine erste Zeitverzögerungsstufe 16 angeschlossen ist, auf die ein Behandlungsstromgenerator 4 folgt, und an den anderen Ausgang eine zweite Zeitverzögerungsstufe 16' angeschlossen ist, auf die ein Behandlungsstromgenerator 4' folgt. Von den Ausgangsstufen der Behandlungsstromgeneratoren 4, 4' führen mechanisch stabile flexible Leitungen 9, 9' zu Einsatzkörpern 10, 10', die in den äußeren Gehörgang der Ohren eines Patienten eingesetzt werden können. Von den Einsatzkörpern 10, 10' führen besonders nachgiebige flexible Leitungen 12, 12' zu Elektroden 11, 11', die zur Kontaktierung der zu stimulierenden Nervenendigungen dienen. Die Verzögerungszeiten der Zeitverzögerungsstufen 16, 16' sind unabhängig voneinander einstellbar und es können so den Elektroden 11 einerseits und den Elektroden 11' andererseits entweder Behandlungsströme zugeführt werden, welche zueinander simultan sind, oder Behandlungsströme, die in Bezug aufeinander zeitlich verschoben sind. In Abwandlung einer zeitlich gleichlaufenden Ansteuerung der Zeitverzögerungsstufen 16, 16' vom Trigger 15 kann man auch nur eine Zeitverzögerungsstufe 16 an den Trigger 15 anschließen und die andere Zeitverzögerungsstufe 16' vom Ausgang der Zeitverzögerungsstufe 16 her ansteuern, wie dies in Fig. 6 strichliert eingezeichnet ist. Analog wie bei der Ausführungsform nach den Figuren 1 bis 4 kann man auch bei der Ausführungsform nach den Figuren 5 und 6 Korrekturstufen 21, 21' vorsehen, mit denen das Ausmaß der Zeitverzögerung in den Zeitverzögerungsstufen 16, 16' beeinflussbar ist.

Anstelle der mechanischen Fixierung des Überganges von den flexiblen Leitungen 9 zu den flexiblen Leitungen 12 mit Hilfe eines Einsatzkörpers 10, der durch Einsetzen in den äußeren Gehörgang einen mechanischen Halt findet, kann man auch andere Lösungen vorsehen, um eine derartige oder ähnliche Fixierung zu erreichen. Als Beispiel hiefür kann man bogenartige um die Ohren zu legende Ohrbügel nennen, welche z.B. in Form eigener Körper ausgebildet sein können oder durch eine entsprechende Biegung der ohrseitigen Enden der flexiblen Leitungen 9 realisiert werden können. Es besteht auch sowohl bei der Ausführungsform nach den Figuren 1 bis 4 als auch bei der Ausführungsform nach den Figuren 5 und 6 die Möglichkeit, anstelle der am Träger 2 vorgesehenen Basiselektrode 13 Basiselektroden vorzusehen, welche in der Nähe der Stimulationselektrode 11 angeordnet werden, wobei hiefür besonders Nadelelektroden in Betracht gezogen werden, welche transkutan in dicht unter der Haut liegende Bereiche eingeführt werden.

Fig. 7 zeigt in Form eines Blockschaltbildes eine vereinfachte Ausführungsform eines erfindungsgemäß ausgebildeten Gerätes, bei dem in einem im Bereich des Halses eines Patienten anzuordnenden Träger 2 eine Speisebatterie 3 und eine Elektronikschaltung angeordnet sind, welche einen hinsichtlich Wellenform-Amplitude und Zeitfaktoren für den Behandlungsstrom programmierbaren Teil 19 des Behandlungsstromgenerators beinhalten, der über eine elektrische Verbindung in Form einer flexiblen Leitung 9 mit einem Einsatzkörper 10 verbunden ist, der als Gehäuse ausgebildet ist und die Ausgangsstufe 18 des Behandlungsstromgenerators sowie eine für deren Speisung vorgesehene Batterie 26 enthält. Vom Einsatzkörper 10 führen flexible Leitungen 12 zu Stimulationselektroden 11 und es ist weiter mindestens eine Basiselektrode 28, die mit einer flexiblen Leitung 29 vom Einsatzkörper 10 ausgeht und vorzugsweise als Nadelelektrode ausgebildet ist, vorgesehen.

Bei der in Figur 8 in Form einer Blockschaltung dargestellten Ausführungsform eines Gerätes 1 ist ein Träger 2 in Form eines Bandes oder einer Manschette vorgesehen. Der Träger 2 nimmt eine Speisebatterie 3 auf und trägt elektronische Schaltkreise 7, welche einen Sensorverstärker 14, einen Trigger 15, eine Zeitverzögerungsstufe 16 und einen Teil 19 des Behandlungsstromgenerators 4 beinhalten, der hinsichtlich Wellenform, Amplitude und Zeitfaktoren des impulsförmigen Behandlungsstromes programmierbar ist. An den Sensorverstärker 14 ist ein Körperfunktionssensor 6 angeschlossen, der z.B. ein Pulssensor oder ein Sensor für elektrische Herzaktionspotentiale sein kann. Wie oben erwähnt, ist der Trigger 15 und die Zeitverzögerungsstufe 16 einstellbar ausgebildet. Die Ausgangsstufe 18 des Behandlungsstromgenerators ist in einem bogenförmig ausgebildeten Gehäuse 27 untergebracht, welches auch eine Speisebatterie 26 für diese Ausgangsstufe enthält und es sind an diese Ausgangsstufe über flexible Leitungen 12 Stimulationselektroden 11 sowie mindestens eine Basiselektrode 28 über eine flexible Leitung 29 angeschlossen. Das bogenförmige Gehäuse 27 kann vorteilhaft in Art eines üblichen Telefon-Head-Sets ausgeführt sein. Zur elektrischen Verbindung des im Träger 2 befindlichen Teiles des Behandlungsstromgenerators mit der im Gehäuse 27 angeordneten Ausgangsstufe 18 dieses Generators ist eine Sender-Empfänger-Strecke S,E vorgesehen, welche vorteilhaft in Bluetoothtechnik ausgeführt sein kann.

Auch bei der in Fig. 7 dargestellten Ausführungsform kann man die elektrische Verbindung zwischen der im Träger 2 angeordneten Elektronikschaltung 19 und der in einem Einsatzkörper 10 angeordneten Ausgangsstufe 18 des Behandlungsstromgenerators gewünschtenfalls in Form einer derartigen Sender-Empfänger-Strecke ausbilden.

## Patentansprüche

1. Gerät zur Punktual-Stimulation von im Bereich der Ohren liegenden Endigungen von zu Hirnstammkernen führenden Nerven, welches Gerät (1) einen batteriegespeisten Behandlungsstromgenerator (4) aufweist, der mit einer einen niederfrequenten Behandlungsstrom formenden Elektronikschaltung versehen ist, und welches Gerät (1) weiter mindestens eine an einer Nervenendigung zu positionierende Elektrode (11) aufweist, die in Form einer kurzen Nadel ausgebildet ist und die über eine eigene flexible Leitung (12) mit dem Behandlungsstromgenerator (4) verbunden ist, **dadurch gekennzeichnet, dass** das Gerät (1) einen in Form einer um den Hals (A) eines Patienten (P) zu legenden Manschette oder Bandes ausgebildeten Träger (2) aufweist, der Speisebatterien (3) aufnimmt, welche mindestens einen Teil der Betriebsenergie des Gerätes (1) liefern, und der weiter mit mindestens einem Teil der für die Bildung des Behandlungsstromes vorgesehenen elektrisch wirksamen Bauteile (4, 7, 13) des Gerätes (1) versehen ist.

2. Gerät nach Anspruch 1, **dadurch gekennzeichnet, dass** mindestens ein Behandlungsstromgenerator (4) an dem in Form einer um den Hals (A) eines Patienten (P) zu legenden Manschette oder Bandes ausgebildeten Träger (2) angeordnet ist.

3. Gerät nach Anspruch 1 oder 2, **dadurch gekennzeichnet, dass** der in Form einer um den Hals (A) eines Patienten (P) zu legenden Manschette oder Bandes ausgebildete Träger (2) mit mindestens einer mit der Hautoberfläche des Patienten in Berührung zu bringenden Basiselektrode (13) versehen ist, über die der über die mindestens eine an einer im Bereich der Ohren eines Patienten liegenden Nervenendigung zu positionierende in Form einer kurzen Nadel ausgebildete Elektrode (11) führende Behandlungsstromkreis geschlossen wird.

4. Gerät nach Anspruch 3, **dadurch gekennzeichnet, dass** mindestens eine als Flächenelektrode ausgebildete Basiselektrode (13) an der mit der Hautoberfläche des Patienten in Berührung zu bringenden Seite des in Form einer um den Hals (A) eines Patienten (P) zu legenden Manschette oder Bandes ausgebildeten Trägers (2) angeordnet ist.

5. Gerät nach einem der Ansprüche 1 bis 4, **dadurch gekennzeichnet, dass** der in Form einer um den Hals (A) eines Patienten (P) zu legenden Manschette oder Bandes ausgebildete Träger (2) mit mindestens einem elektrische Ausgangssignale abgebenden Sensor (6) für Körperfunktionen eines Patienten versehen ist, weiter elektronische Schaltkreise (7) aufweist, die aus dem Ausgangssignal des Körperfunktionssensors (6) Steuersignale bilden, welche für eine von den Körperfunktionen abhängige Steuerung mindestens eines Behandlungsstromgenerators (4)vorgesehen sind, und weiter eine zu mindestens einem Behandlungsstromgenerator (4) führende elektrische Signalverbindung zur Beeinflussung des Behandlungsstromes durch die von den Ausgangssignalen des Sensors (6) hergeleiteten Steuersignale vorgesehen ist.

6. Gerät nach Anspruch 5, **dadurch gekennzeichnet, dass** der in Form einer um den Hals (A) eines Patienten (P) zu legenden Manschette oder Bandes ausgebildete Träger (2) mit einem Pulssensor versehen ist.

7. Gerät nach Anspruch 5 oder 6, **dadurch gekennzeichnet, dass** der in Form einer um den Hals (A) eines Patienten (P) zu legenden Manschette oder Bandes ausgebildete Träger (2) mit einem Sensor (6) für elektrische Herzaktionspotentiale versehen ist.

8. Gerät nach einem der Ansprüche 5 bis 7, **dadurch gekennzeichnet, dass** der in Form einer um den Hals (A) eines Patienten (P) zu legenden Manschette oder Bandes ausgebildete Träger (2) mit einem Sensor für Hirnströme versehen ist.

9. Gerät nach Anspruch 5, **dadurch gekennzeichnet, dass** die elektronischen Schaltkreise (7), die aus dem Ausgangssignal des Körperfunktionssensors (6) Steuersignale für den Behandlungsstromgenerator (4) bilden, eine vom periodischen Ausgangssignal eines Körperfunktionssensors (6) triggerbare, hinsichtlich ihrer Verzögerungszeit einstellbare Zeitverzögerungsstufe (16) aufweisen, wobei die Zeitverzögerungsstufe (16) ihrerseits den Behandlungsstromgenerator (4) ansteuert, welcher an mindestens eine zur Platzierung an einer im Bereich des äußeren Ohres eines Patienten liegenden Nervenendigung bestimmte in Form einer kurzen Nadel ausgebildete Elektrode (11) einen periodischen Behandlungsstrom abgibt, dessen Folgefrequenz der Folgefrequenz des Ausgangssignals des Körperfunktionssensors (6) entspricht und der aus Stromflusspaketen gebildet ist, deren Länge geringer ist, als die Periodendauer des Ausgangssignals des Körperfunktionssensors, wobei die elektronischen Schaltkreise (7) eine Korrekturstufe (21) beinhalten, welche aus dem Frequenzwert oder aus der Kurvenform oder aus dem Amplitudenwert des Ausgangssignals des Körperfunktionssensors (6) ein Korrektursignal bildet, das an einen die Zeitverzögerung beeinflussenden Eingang (22) der Zeitverzögerungsstufe (16) angeschlossen ist.

10. Gerät nach Anspruch 9, **dadurch gekennzeichnet, dass** an die Zeitverzögerungsstufe (16) ein Verschiebungssignalgeber (24) angeschlossen ist, der der Zeitverzögerungsstufe (16) ein Schiebesignal zuführt, durch das die Verzögerungszeit, im Verhältnis zur Folgefrequenz des Ausgangssignals des Körperfunktionssensors (6) langsam, veränderbar ist.

11. Gerät nach einem der Ansprüche 1 bis 10, **dadurch gekennzeichnet, dass** das Gerät (1) mindestens eine an einer Nervenendigung im Bereich des äußeren Ohres zu positionierende Elektrode (11) aufweist, die in Form einer kurzen Nadel ausgebildet ist und deren flexible Leitung (12) von einem passend in den äußeren Gehörgang einfügbaren Einsatzkörper (10) ausgeht, der seinerseits in elektrischer Verbindung mit am in Form einer um den Hals (A) eines Patienten (P) zu legenden Manschette oder Bandes ausgebildeten Träger (2) befindlichen Teilen des Gerätes steht.

12. Gerät nach Anspruch 11, **dadurch gekennzeichnet, dass** der Einsatzkörper (10) als Gehäuse ausgebildet ist, in dem mindestens der den Behandlungsstrom an die in Form einer kurzen Nadel ausgebildete Elektrode (11) abgebende Teil (18) der Elektronikschaltung des Behandlungsstromgenerators (4) angeordnet ist.

13. Gerät nach Anspruch 12, **dadurch gekennzeichnet, dass** mindestens ein Behandlungsstromparameter, wie z.B. das Stromfluss-Pausen-Programm oder die Frequenz, die Amplitude und die Kurvenform des Behandlungsstromes formender Teil (19) der Elektronikschaltung (5) des Behandlungsstromgenerators (4) in dem in Form einer um den Hals (A) eines Patienten (P) zu legenden Manschette oder Bandes ausgebildeten Träger (2) angeordnet ist und über eine elektrische Verbindung den im Einsatzkörper (10) befindlichen Teil (18) der Elektronikschaltung des Behandlungsstromgenerators (4) steuert.

14. Gerät nach einem der Ansprüche 1 bis 10, **dadurch gekennzeichnet, dass** das Gerät mindestens eine an einer Nervenendigung im Bereich des äußeren Ohres zu positionierende in Form einer kurzen Nadel ausgebildete Elektrode (11) aufweist, deren flexible Leitung (12) von einem außen am Ohr zu tragenden Gehäuse (27) ausgeht, in dem mindestens der den Behandlungsstrom an die Elektrode abgebende Teil (18) der Elektronikschaltung des Behandlungsstromgenerators (4) angeordnet ist, und dass mindestens ein Behandlungsstromparameter, wie z.B. das Stromfluss-Pausen-Programm oder die Frequenz, die Amplitude und die Kurvenform des Behandlungsstromes formender Teil (19) der Elektronikschaltung des Behandlungsstromgenerators (4) in dem in Form einer um den Hals (A) eines Patienten (P) zu legenden Manschette oder Bandes ausgebildeten Träger (2) angeordnet ist und über eine elektrische Verbindung den im am Ohr zu tragenden Gehäuse (27) befindlichen Teil (18) der Elektronikschaltung des Behandlungsstromgenerators (4) steuert.

15. Gerät nach einem der Ansprüche 11 bis 13, **dadurch gekennzeichnet, dass** mindestens eine Nadelelektrode (28) vorgesehen ist, die über eine flexible Leitung (29) am Einsatzkörper (10) angeordnet und mit dem Behandlungsstromgenerator (4) verbunden ist und eine Basiselektrode bildet, über die ein Behandlungsstromkreis geschlossen wird, der über eine an einer Nervenendigung zu positionierende in Form einer kurzen Nadel ausgebildete Elektrode (11) führt.

16. Gerät nach Anspruch 14, **dadurch gekennzeichnet, dass** mindestens eine Nadelelektrode (28) vorgesehen ist, die über eine flexible Leitung (29) an dem außen am Ohr zu tragenden Gehäuse (27) angeordnet und mit dem Behandlungsstromgenerator (4) verbunden ist und eine Basiselektrode bildet, über die ein Behandlungsstromkreis geschlossen wird, der über eine an einer Nervenendigung zu positionierende in Form einer kurzen Nadel ausgebildete Elektrode (11) führt.

## Claims

1. A device for point stimulation of the ends of nerves in the vicinity of the ears, the nerve ends leading to brain stem cores, the device (1) comprising a battery-driven treatment current generator (4) provided with an electronic circuit which forms a low-frequency treatment current, the device (1) further comprising at least one electrode (11) that is to be positioned at a nerve ending, the electrode being designed in the shape of a short needle and being connected to the treatment current generator (4) by way of an integral flexible line (12), **characterized in that** the device (1) comprises a support (2) in the form of a collar or strap placed around the neck (A) of a patient (P), the support holding the drive batteries (3) which provide at least a portion of the operating energy of the device (1), the support also being provided with at least some of the components (4, 7, 13) of the device (1) responsible for generating the treatment current.

2. A device according to claim 1, **characterized in that** at least one treatment current generator (4) is disposed at the support (2) designed in the form of a collar or strap placed around the neck (A) of a patient (P).

3. A device according to claim 1 or 2, **characterized in that** the support (2) designed in the form of a collar or strap placed around the neck (A) of a patient (P) is provided with at least one base electrode (13) which is to be brought into contact with the skin surface of the patient, the treatment current circuit passing through the at least one electrode (11) designed in the form of a short needle and positioned at a nerve ending located near the ear of a patient, the circuit being closed via the base electrode.

4. The device according to claim 3, **characterized in that** at least one base electrode (13) designed as a flat electrode is placed on the side of the support (2), which is designed in the form of a collar or strap placed around the neck (A) of a patient (P), that is to be brought into contact with the skin surface of the patient.

5. The device according to one of claims 1 to 4, **characterized in that** the support (2) designed in the form of a collar or strap placed around the neck (A) of a patient (P) is provided with at least one patient body function sensor (6) that issues electrical output signals, the support also comprising electronic circuits (7) that generate control signals from the output signal of the body function sensor (6), the control signals being provided to control at least one treatment current generator (4) as a function of the body functions, and also comprising an electric signal connection for influencing the treatment current by way of the control signals that are derived from the output signals of the sensor (6), said connection leading to at least one treatment current generator (4).

6. The device according to claim 5, **characterized in that** the support (2) designed in the form of a collar or strap placed around the neck (A) of a patient (P) is provided with a pulse sensor.

7. The device according to claim 5 or 6, **characterized in that** the support (2) designed in the form of a collar or strap placed around the neck (A) of a patient (P) is provided with a sensor (6) for electric heart action potentials.

8. The device according to one of claims 5 to 7, **characterized in that** the support (2) designed in the form of a collar or strap placed around the neck (A) of a patient (P) is provided with a brainwave sensor (2).

9. The device according to claim 5, **characterized in that** the electronic circuits (7) that generate control signals for the treatment current generator (4) from the output signal of the body function sensor (6) comprise a time delay step (16) that can be triggered by the periodic output signal of a body function sensor (6), the delay time of said delay step being adjustable, wherein the time delay step (16) in turn controls the treatment current generator (4), the generator issuing a periodic treatment current to at least one electrode (11), designed in the form of a short needle and intended to be placed at a nerve ending near the outer ear of a patient, the repetition frequency of the periodic treatment current matching the repetition frequency of the output signal of the body function sensor (6) and being made up of packets of flowing current that are of a shorter length than the period duration of the output signal of the body function sensor, wherein the electronic circuits (7) include a correction stage (21) that generates a correction signal from the frequency value from the curve shape or from the amplitude value of the output signal of the body function sensor (6), the correction signal being connected to an input (22) of the time delay step (16), the input influencing the time delay.

10. The device according to claim 9, **characterized in that** a shift signal transmitter (24) is connected to the time delay step (16), the transmitter feeding a shift signal to the time delay step (16), the shift signal being able to change the delay time slowly relative to the repetition frequency of the output signal of the body function sensor (6).

11. The device according to one of claims 1 to 10, **characterized in that** the device (1) comprises at least one electrode (11) positioned at a nerve ending near the outer ear, the electrode being designed in the form of a short needle with a flexible line (12) issuing from a fitted insertion element (10) that can be inserted into the outer ear canal, the insertion element in turn being in electrical connection with parts of the device that are on the support (2) designed in the form of a collar or strap placed around the neck (A) of a patient (P).

12. The device according to claim 11, **characterized in that** the insertion element (10) is designed as a housing inside of which is located at least the part (18) of the electronic circuit of the treatment current generator (4) that issues the treatment current to the electrode (11) designed in the form of a short needle.

13. The device according to claim 12, **characterized in that** at least one part (19) of the electronic circuit (5) of the treatment current generator (4) generates treatment current parameters, such as the current flow pause program or the frequency, amplitude and curve shape of the treatment current, said part being located in the support (2) designed in the form of a collar or strap placed around the neck (A) of a patient (P) and controlling, by way of an electrical connection, the part (18) of the electronic circuit of the treatment current generator (4) that is located inside the insertion element (10).

14. The device according to one of claims 1 to 10, **characterized in that** the device comprises at least one electrode (11) designed in the form of a short needle and positioned at a nerve ending near the outer ear, the flexible line (12) of said electrode issuing from a housing (27) worn on the outside at the ear, inside of said housing is located at least the part (18) of the electronic circuit of the treatment current generator (4) that issues the treatment current to the electrode, and that at least one part (19) of the electronic circuit of the treatment current generator (4) generates treatment current parameters, such as the current flow pause program or the frequency, the amplitude and the curve shape of the treatment current, said part being located in the support (2) designed in the form of a collar or strap placed around the neck (A) of a patient (P) and controlling, by way of an electrical connection, the part (18) of the electronic circuit of the treatment current generator (4) that is located inside the housing (27) worn at the ear.

15. The device according to one of claims 11 to 13, **characterized in that** at least one needle electrode (28) is provided, said electrode located at the insertion member (10) by way of a flexible line (29) and being connected to the treatment current generator (4) and constituting a base electrode for closing a treatment current circuit that passes through an electrode (11) designed in the form of a short needle and positioned at a nerve ending.

16. The device according to claim 14, **characterized in that** at least one needle electrode - (28) is provided at the outer housing (27) worn on the outside at the ear, said electrode being connected to the treatment current generator (4) by way of a flexible line (29) and constituting a base electrode for closing a treatment current circuit that passes through an electrode (11) designed in the form of a short needle and positioned at a nerve ending.

## Revendications

1. - Appareil de stimulation ponctuelle des terminaisons nerveuses, situées dans la région de l'oreille, des nerfs conduisant aux noyaux du tronc cérébral, ledit appareil (1) comprenant un générateur de courant de traitement (4) alimenté par piles qui comporte un circuit électronique de formation d'un courant de traitement à basse fréquence, ledit appareil (1) comprenant en outre au moins une électrode (11) qui est destinée à être mise en place sur une terminaison nerveuse, qui prend la forme d'une aiguille courte et qui est reliée par sa propre ligne flexible (12) au générateur de courant de traitement (4), **caractérisé en ce que** l'appareil (1) possède un support (2) prenant la forme d'un manchon ou d'une courroie à poser autour du cou (A) d'un patient (P), qui reçoit des piles d'alimentation (3) qui fournissent au moins une partie de l'énergie de fonctionnement de l'appareil (1) et qui comporte en outre au moins une partie des composants (4, 7, 13) de l'appareil (1) électriquement actifs qui sont destinés à former le courant de traitement.

2. - Appareil selon la revendication 1, **caractérisé en ce qu'**au moins un générateur de courant de traitement (4) est disposé sur le support (2) prenant la forme d'un manchon ou d'une courroie à poser autour du cou (A) d'un patient (P).

3. - Appareil selon l'une des revendications 1 ou 2, **caractérisé en ce que** le support (2) prenant la forme d'un manchon ou d'une courroie à poser autour du cou (A) d'un patient (P) comporte au moins une électrode de base (13) devant être amenée au contact de la surface de la peau du patient, grâce à laquelle se trouve fermé le circuit de courant de traitement qui passe par ladite au moins une électrode (11) à mettre en place sur une terminaison nerveuse dans la région de l'oreille d'un patient et prenant la forme d'une aiguille courte.

4. - Appareil selon la revendication 3, **caractérisé en ce qu'**au moins une électrode de base (13) prenant la forme d'une électrode plate est disposée sur la face du support (2) prenant la forme d'un manchon ou d'une courroie à poser autour du cou (A) d'un patient (P) qui doit être amenée au contact de la surface de la peau du patient.

5. - Appareil selon l'une des revendications 1 à 4, **caractérisé en ce que** le support (2) prenant la forme d'un manchon ou d'une courroie à poser autour du cou (A) d'un patient (P) est doté d'au moins un détecteur (6) de fonctions corporelles d'un patient qui fournit des signaux électriques de sortie, possède en outre des circuits électroniques (7) qui forment à partir des signaux de sortie du détecteur (6) de fonctions corporelles des signaux de commande qui sont destinés à une commande, qui dépend des fonctions corporelles, d'au moins un générateur de courant de traitement (4) et comporte en outre une liaison électrique de signaux conduisant à au moins un générateur de courant de traitement (4) afin que les signaux de commande dérivés des signaux de sortie du détecteur (6) affectent le courant de traitement.

6. - Appareil selon la revendication 5, **caractérisé en ce que** le support (2) prenant la forme d'un manchon ou d'une courroie à poser autour du cou (A) d'un patient (P) comporte un capteur de rythme cardiaque.

7. - Appareil selon l'une des revendications 5 ou 6, **caractérisé en ce que** le support (2) prenant la forme d'un manchon ou d'une courroie à poser autour du cou (A) d'un patient (P) comporte un capteur (6) de potentiels électriques d'action cardiaque.

8. - Appareil selon l'une des revendications 5 à 7, **caractérisé en ce que** le support (2) prenant la forme d'un manchon ou d'une courroie à poser autour du cou (A) d'un patient (P) comporte un capteur de courants cérébraux.

9. - Appareil selon la revendication 1, **caractérisé en ce que** les circuits électroniques (7) qui forment à partir du signal de sortie du détecteur de fonctions corporelles (6) des signaux de commande pour le générateur de courant de traitement (4) possèdent un étage de temporisation (16), à temporisation réglable, qui peut être déclenché par le signal périodique de sortie d'un détecteur (6) de fonctions corporelles, l'étage de temporisation (16) pilotant à son tour le générateur de courant de traitement (4) qui fournit à au moins une électrode (11) à mettre en place sur une terminaison nerveuse se situant dans la région de l'oreille externe d'un patient et prenant la forme d'une aiguille courte un courant périodique de traitement dont la fréquence de répétition correspond à la fréquence de répétition du signal de sortie du détecteur (6) de fonctions corporelles et qui est formé de paquets de courant dont la longueur est inférieure à la durée de la période du signal de sortie du détecteur de fonctions corporelles, les circuits électroniques (7) possédant un étage de correction (21) qui, à partir de la valeur de la fréquence ou de l'allure ou de la valeur de l'amplitude du signal de sortie du détecteur (6) de fonctions corporelles, produit un signal de correction qui est raccordé à une entrée (22) de l'étage de temporisation (16) qui affecte la temporisation.

10. - Appareil selon la revendication 9, **caractérisé en ce qu'**à l'étage de temporisation (16) est raccordé un codeur de signal de décalage (24) qui fournit à l'étage de temporisation (16) un signal de décalage grâce auquel il est possible de faire varier lentement la durée de temporisation, par rapport à la fréquence de répétition du signal de sortie du détecteur (6) de fonctions corporelles.

11. - Appareil selon l'une des revendications 1 à 10, **caractérisé en ce que** l'appareil (1) possède au moins une électrode (11) à mettre en place sur une terminaison nerveuse dans la région de l'oreille externe, qui prend la forme d'une aiguille courte et dont la ligne flexible (12) part d'un corps d'insert (10) qui peut être introduit de manière adéquate dans le conduit auriculaire extérieur et qui est à son tour en liaison électrique avec des parties de l'appareil qui se situent sur le support (2) prenant la forme d'un manchon ou d'une courroie à poser autour du cou (A) d'un patient (P).

12. - Appareil selon la revendication 11, **caractérisé en ce que** le corps d'insert (10) prend la forme d'un boîtier dans lequel se situe au moins la partie (18) du circuit électronique du générateur de courant de traitement (4) qui fournit le courant de traitement à l'électrode (11) prenant la forme d'une aiguille courte.

13. - Appareil selon la revendication 12, **caractérisé en ce qu'**au moins une partie (19) du circuit électronique (5) du générateur de courant de traitement (4) qui forme des paramètres du courant de traitement, tels que, par exemple, le programme de courant et de pauses ou la fréquence, l'amplitude et l'allure du courant de traitement, est située dans le support (2) prenant la forme d'un manchon ou d'une courroie à poser autour du cou (A) d'un patient (P) et commande par une liaison électrique la partie (18) du circuit électronique du générateur de courant de traitement (4) qui se situe dans le corps d'insert (10).

14. - Appareil selon l'une des revendications 1 à 10, **caractérisé en ce que** l'appareil possède au moins une électrode (11) à mettre en place sur une terminaison nerveuse dans la région de l'oreille externe et prenant la forme d'une aiguille courte, dont la ligne flexible (12) sort d'un boîtier (27) qui est à porter extérieurement sur l'oreille et dans lequel est disposée au moins la partie (18) du circuit électronique du générateur de courant de traitement (4) qui fournit le courant de traitement à l'électrode, et **en ce qu'**au moins une partie (19) du circuit électronique du générateur de courant de traitement (4) qui forme des paramètres du courant de traitement, tels que, par exemple, le programme de courant et de pauses ou la fréquence, l'amplitude et l'allure du courant de traitement, est située dans le support (2) prenant la forme d'un manchon ou d'une courroie à poser autour du cou (A) d'un patient (P) et commande par une liaison électrique la partie (18) du circuit électronique du générateur de courant de traitement (4) qui se situe dans le boîtier (27) à porter sur l'oreille.

15. - Appareil selon l'une des revendications 11 à 13, **caractérisé en ce qu'**il est prévu au moins une électrode-aiguille (28) qui est disposée sur le corps d'insert (10) par l'intermédiaire d'une ligne flexible (29), qui est reliée au générateur de courant de traitement (4) et qui forme une électrode de base grâce à laquelle est fermé un circuit de courant de traitement qui passe par une électrode (11) à mettre en place sur une terminaison nerveuse et prenant la forme d'une aiguille courte.

16. - Appareil selon la revendication 14, **caractérisé en ce qu'**il est prévu au moins une électrode-aiguille (28) qui est disposée sur le boîtier (27) à porter extérieurement sur l'oreille par l'intermédiaire d'une ligne flexible (29), qui est reliée au générateur de courant de traitement (4) et qui forme une électrode de base grâce à laquelle est fermé un circuit de courant de traitement qui passe par une électrode (11) à mettre en place sur une terminaison nerveuse et prenant la forme d'une aiguille courte.
